# EUROPEAN PATENT APPLICATION

(11) **EP 4 729 945 A1**
(43) Date of publication of application: **22.04.2026**
(21) Application number: 25208945.3
(22) Date of filing: 15.10.2025
(51) Int. Cl.: G01N 33/5755, G01N 33/74

(54) **BIOMARKER SET AND IN VITRO METHOD FOR DIAGNOSING ENDOMETRIAL CANCER OF THE UTERINE CORPUS**

(30) Priority: 15.10.2024 PL 45003424
(71) Applicant: Gdanski Uniwersytet Medyczny, 80-210 Gdansk (PL); Uniwersytet Gdanski, 80-309 Gdansk (PL)
(72) Inventor: Gorska-Ponikowska, Magdalena, 80-442 Gdansk (PL); Kostrzewa, Tomasz, 76-200 Slupsk (PL); Sawicki, Sambor, 80-293 Gdansk (PL); Bukato, Katarzyna, 80-414 Gdansk (PL); Jacewicz, Dagmara, 80-134 Gdansk (PL); Konieczna, Lucyna, 84-240 Reda (PL); Baczek, Tomasz, 80-174 Otomin (PL); Drzezdzon, Joanna, 84-242 Luzino (PL)
(74) Representative: JD&P Patent Attorneys Joanna Dargiewicz & Partners

(57) **Abstract**

The subject of the invention is a set of biomarkers containing estradiol, 2-methoxyestradiol, and hydrogen peroxide for use in the in vitro diagnosis of endometrial cancer in an individual, and a method for the in vitro diagnosis of endometrial cancer in an individual.

## Description

The subject of the invention is a set of three biomarkers, namely estradiol (E2), 2-methoxyestradiol (2-MeOE2) and hydrogen peroxide (H₂O₂), especially in the reactive form of oxygen, for use in the *in vitro* diagnosis of endometrial cancer, and a method for the *in vitro* diagnosis of endometrial cancer. The invention relates to the field of oncological diagnostics.

Endometrial cancer is the most common gynecological cancer among women in Poland and worldwide. Starting treatment at an early stage gives patients a chance for a complete cure and avoids aggressive therapeutic methods that often impair quality of life. Currently, there are no proven biomarkers for the diagnosis of endometrial cancer that would assess the risk of the disease and enable effective, widespread screening. Despite the great progress that has been made in cancer diagnosis over the past few decades, progress in the diagnosis of endometrial cancer has been very limited, if any. There are a number of publications describing diagnostic methods for endometrial cancer, but they are mainly based on clinical symptoms and ultrasound imaging [1-4]. The most common symptom of a proliferative process is abnormal postmenopausal bleeding (PMB), which is an indication for further diagnosis involving the collection of a biopsy or curettage from the uterine cavity. According to the available literature, more than 90% of patients who experience bleeding will not be diagnosed with cancer, and this group of women is unnecessarily exposed to possible complications associated with invasive diagnostics [5]. Therefore, the development of non-invasive methods for early detection of the disease is of great importance, which has led to a steady increase in interest in the search for sensitive and specific biomarkers.

Estrogens and their metabolites are a fundamental risk factor for endometrial cancer and play a key role in its pathogenesis [6-7]. An imbalance in their concentrations affects the expression of genes involved in cell proliferation and apoptosis [8]. A number of studies have been conducted on the pro- and anti-cancer properties of estrogen metabolites, but due to unclear results, they have not been used in clinical practice as a diagnostic tool [9-12]. To so far, the best-proven effects appear to be the procarcinogenic action of estradiol and the protective properties of 2-methoxyestradiol [13-16].

In addition, estrogen metabolites may contribute to the formation of reactive oxygen species (ROS) [17]. Excessive production of ROS and depletion of antioxidant reserves by the body is a phenomenon called "oxidative stress," which in cancer processes should be considered both as an initiator of cancer progression, as an element promoting cancer progression, and as a factor responsible for cell cycle arrest and apoptosis of cancer cells.

Because blood is readily available and its use is generally accepted by both clinicians and patients, it is a good source of biomarkers. New advances in metabolomics have enabled the identification and quantification of a wide range of metabolites. At the current state of the art, diagnostic methods for the determination of tumor markers play only a supporting role due to insufficient sensitivity, specificity, and predictive value. These markers are more widely used in the treatment process and during long-term follow-up of patients after treatment. There is also a lack of biomarkers with proven efficacy for routine detection of endometrial cancer.

In order to increase the sensitivity and specificity of the proposed screening test, the inventors additionally assessed the level of oxidative stress by measuring hydrogen peroxide concentration in addition to measuring estrogen metabolite concentrations. In the course of the experimental studies, it was found that biomarkers based on estrogen metabolites from different pathways in correlation with the assessment of hydrogen concentration may be useful for identifying women at high risk of developing this disease.

The subject of the invention is a set of biomarkers containing estradiol (E2), 2-methoxyestradiol (2-MeOE2), and hydrogen peroxide (H₂O₂) for use in the *in vitro* diagnosis of endometrial cancer in an individual.

Another subject of the invention is a method for *in vitro* diagnosis of endometrial cancer in an individual, characterized in that it comprises the following steps:
a). In a biological sample taken from an individual, the level of at least two biomarkers selected from a set comprising estradiol, 2-methoxyestradiol, and hydrogen peroxide is determined.
b). The levels of biomarkers in a) shall be assessed and compared with reference samples,
whereby endometrial cancer is diagnosed when there is a twofold increase in the level of estradiol and a twofold decrease in the level of 2-methoxyestradiol compared to the reference sample taken from a healthy individual, and an increased level of hydrogen peroxide compared to the reference sample.

Advantageously, in the method according to the invention, the biological sample is blood plasma.

Advantageously, in the method according to the invention, the levels of estradiol and 2-methoxyestradiol are determined by liquid chromatography coupled with tandem mass spectrometry (LC-MS/MS).

Advantageously, in the method according to the invention, the level of hydrogen peroxide is determined by the stopped-flow method.

Advantageously, in the method according to the invention, an elevated level of hydrogen peroxide is a concentration of hydrogen peroxide in the range of 5.93 ± 2.46 µmol/L.

Advantageously, in the method according to the invention, the level of estradiol is at least twice as high as the level of 2-methoxyestradiol in an individual suffering from endometrial cancer.

The set of biomarkers and the method for *in vitro* diagnosis of endometrial cancer, which is the subject of the invention, involves the use of biomarkers and the preparation of biological samples obtained from the patient, allowing for the detection of significant changes and correlations in the concentrations of estrogen metabolites, enabling more effective and non-invasive early detection of endometrial cancer. This will make it possible to screen a wider group of women without exposing them to the possible complications of the diagnostic method used to date, i.e., the collection of material from the uterine cavity. It will also allow for earlier detection of the disease in patients who have not experienced abnormal postmenopausal bleeding, despite the ongoing proliferative process. This method is more acceptable to patients. In the course of further research, this method could be used not only for diagnosis, but also for monitoring patients with endometrial cancer.

The subject of the invention is presented by means of the figures in the drawings, of which:
Fig. 1 shows how to prepare plasma samples for analysis

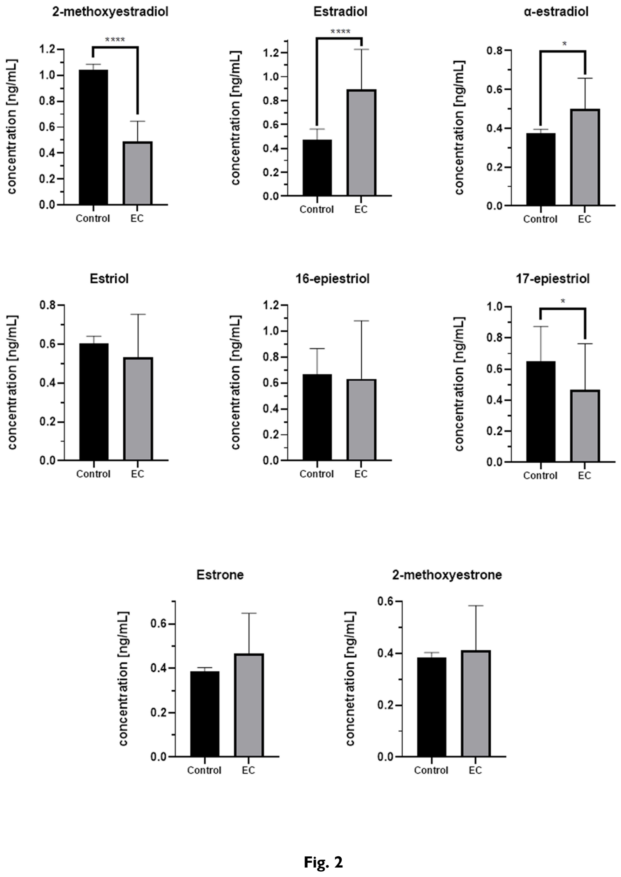

Fig. 2 shows the analysis between the control group and patients with endometrial cancer (EC) of a given metabolite, statistical test: Mann-Whitney U test (* p < 0.05, **** p < 0.0001)

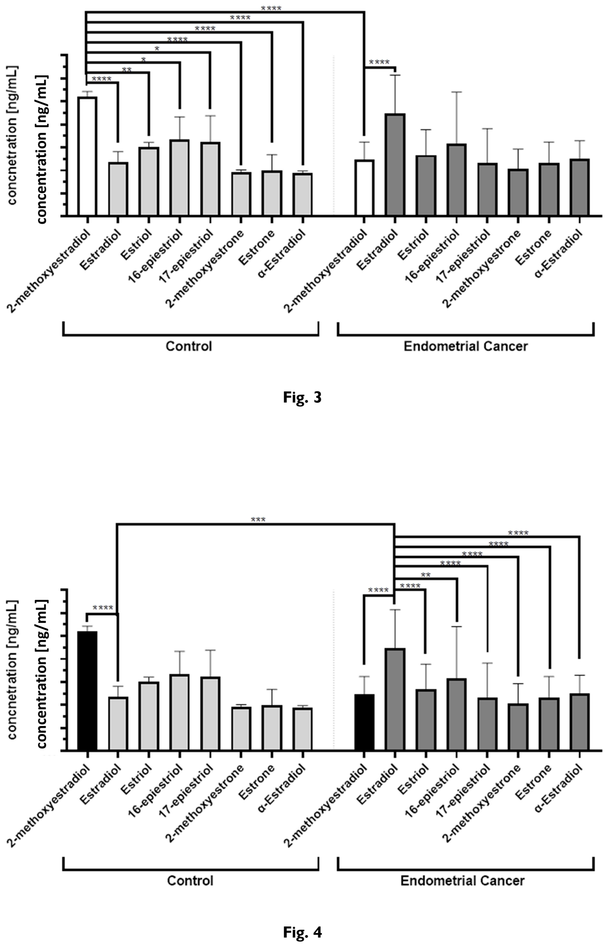

Fig. 3 shows the analysis of the ratio of estradiol to other metabolites, one-way ANOVA, with Tukey's correction - (* p < 0.05, ** p < 0.01, *** p < 0.001, **** p <0.0001)
Fig. 4 presents an analysis of the ratio of 2-methoxyestradiol to other metabolites, one-way ANOVA, with Tukey's correction - (* p < 0.05, ** p < 0.01, *** p < 0.001, **** p < 0.0001)

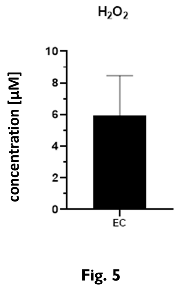

Fig. 5 shows the analysis between the control group and patients with endometrial cancer (EC) hydrogen peroxide, statistical test: Mann-Whitney test.

The invention is illustrated by the following embodiments.

### Embodiments

### Example 1

### A. Testing of estrogen metabolite levels using LC-MS

The first stage of the study involved assessing the concentrations of individual estrogen metabolites in the serum of patients with cancer of the endometrium. For this purpose, 30 patients (n = 30) aged 35-87 years with diagnosed endometrial cancer were enrolled in the study. The control group consisted of 10 women (n = 10), volunteers aged 52-84, who were qualified for the study by a gynecologist after exclusion of an active proliferative process in the uterine cavity. The criterion for inclusion in the control group was a histopathological result of scrapings from the uterine cavity excluding a proliferative process or an unambiguous ultrasound image. Blood for testing was collected before surgical treatment and before the start of chemotherapy, using sodium heparin. The blood was then centrifuged at 1200 rpm for 10 minutes to separate the plasma from the red blood cells. The plasma was stored at -85°C until analysis.

Quantitative determination of estrogens and their hydroxy and methoxy derivatives in biological samples was performed using liquid chromatography coupled with tandem mass spectrometry (LC-MS/MS) using a Shimadzu 8050 device (Kyoto, Japan). The HPLC system consists of: an automatic sample feeder (Autosampler Nexera XR SIL-20AC XR), a two-way pump (Bin Pum Nexera XR LC-30AD XR), a thermostat (CTO-20AC), a Poroshell 120 EC-C18 2.7 µm with dimensions of 3.0 x 100 mm (Agilent Technologies, Santa Clara, USA),

### HPLC conditions

Chromatographic separation was performed using a Poroshell 120 EC-C18 column (dimensions 3.0 x 100 mm; 2.7 µm), thermostated at 40°C. Mobile phase A consisted of water with 0.1% formic acid added, while phase B was methanol. The volumetric flow rate of the mobile phase was 0.3 mL/min. Separation of the analytes was performed by gradient elution with a gradient duration of 47 minutes, where the content of phase B increased from 72% to 87% between 0 and 45 minutes, and then from 87% to 100% between 45 and 47 minutes. In the next stage, column equilibration lasted 8 minutes and the proportion of phase B decreased to 72%. The total time of a single analysis was 55 minutes.

Chromatographic analysis was preceded by the isolation of analytes from biological material by liquid-liquid extraction using dichloromethane.

### Mass detector operating conditions

The MS/MS spectrometer used electrospray ionization (ESI) and a triple quadrupole analyzer. The mass detector operated in positive ionization mode. The analysis conditions for the MS detector were set as follows: nebulizing gas (nitrogen) flow rate 3 L/min, heating gas (nitrogen) was 10 L/min, drying gas (nitrogen) was 10 L/min, the temperature of the electrospray ionization (ESI) source was 300°C, the desolvation line temperature was 250°C, and the heating block temperature was 400°C. Based on the recorded signal intensities, a voltage of 3 kV was applied to the capillary. The volume of the injected sample was 3 µL.

Table 1 shows the optimization results for all standards analyzed by LC-MS/MS. All estrogen derivatives analyzed were identified and quantified by monitoring the corresponding transitions from parent ions to daughter ions in multiple reaction monitoring (MRM) mode, as shown in Table 1.

**Table 1 - MS/MS optimization results for the analysis of estrogen derivatives**

| *No.* | *Estrogen derivative* tested | *Parent ion [M+H]⁺ m*/*z* | *Daughter ion m*/*z* | *CE [V]* | *Monoisotopic mass [Da]* | *Mass of analytes after derivatization [g*/*mol]* |
|---|---|---|---|---|---|---|
| *1* | E₁ | 504.000 | 171.300 | -32 | 270.161 | 504 |
| | | | 156.100 | -54 | | |
| | | | 143.000 | -35 | | |
| *2* | aE₂ | 506.000 | 171.000 | -35 | 272.177 | 506 |
| | | | | -40 | | |
| | | | | -45 | | |
| *3* | E₂ | 506.500 | 171.100 | -36 | 272.177 | 506 |
| | | | 156.000 | -55 | | |
| | | | 170.200 | -46 | | |
| *4* | E3 | 522.500 | 171.100 | -36 | 288.172 | 522 |
| | | | 156.200 | -50 | | |
| | | | 391.000 | -20 | | |
| *5* | 16-epiE₃ | 522.500 | 171,250 | -37 | 288.172 | 522 |
| | | | 156.100 | -53 | | |
| | | | 280.100 | -20 | | |
| *6* | 2-MeOE₁ | 534.200 | 171.100 | -34 | 300.172 | 534 |
| | | | 156.250 | -50 | | |
| | | | 428.000 | -16 | | |
| *7* | 2-MeOE₂ | 536.000 | 171.000 | -37 | 302.188 | 536 |
| | | | | -40 | | |
| | | | | -30 | | |
| *8* | D₄-E₂ | 510.000 | 171.000 | -35 | 276.202 | 510 |
| | | | | -30 | | |
| | | | | -20 | | |

### Preparation of plasma samples for analysis

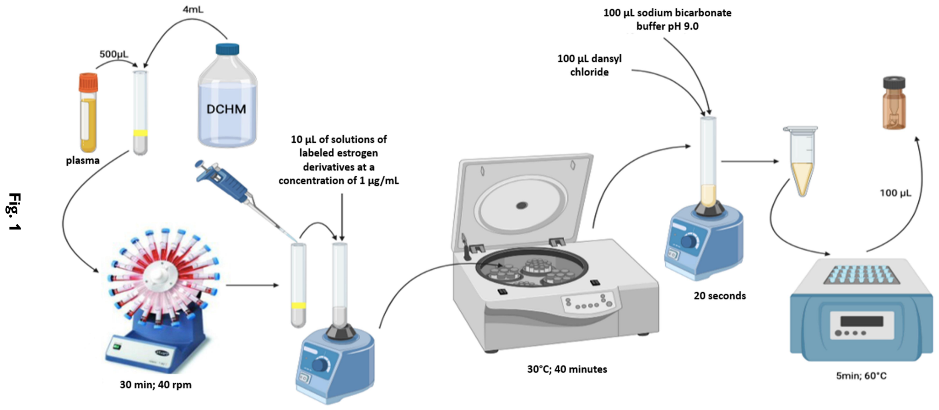

500 µL was taken from the obtained plasma samples and transferred to glass test tubes. Then, 4 mL of dichloromethane was added. They were placed in a rotary shaker and the contents were mixed for 30 minutes at a speed of 40 rpm. After this time, the lower organic layer was collected and transferred to new glass tubes. Each tube was given 10 µL of a labeled internal standard solution (β-estradiol-d₄ ) at a concentration of 1 µg/mL was added to each tube, mixed on a single-point shaker, and evaporated to dryness under vacuum conditions at 30°C for 40 minutes in a CentriVap vacuum concentrator (Labconco, Kansas City, USA). To the dry residue, 100 µL of 0.1M sodium bicarbonate buffer pH 9.0 and 100 µL of dansyl chloride were added to derivatize the analytes, which resulted in an increase in the mass of the analyzed ions, as shown in Table 1. The contents of the test tube were mixed for 20 seconds on a single-point shaker and transferred to an Eppendorf tube. The sample was heated for 5 minutes at 60°C in a heating block with shaking capability. One hundred µL of the solution of derivatized compounds was taken and transferred to 150 µL inserts placed in vials, which were then transferred to an automatic sample feeder. The samples prepared in this way (Fig. 1) were analyzed by LC-MS/MS under the conditions described above.

The results of the measurements are presented in Table 2.

**Table 2 - Results of the analysis of estrogen derivative concentrations in a group of patients with endometrial cancer and in a control group.**

| *No.* | *Estrogen derivative tested* | *Group with endometrial cancer [n=30]* | | *Control group [n=10]* | |
|---|---|---|---|---|---|
| | | MEDIAN *[ng*/*mL]* | SD | MEDIAN *[ng*/*mL]* | SD |
| *1* | E₁ | 0.466 | 0.181 | 0.401 | 0.135 |
| *2* | αE₂ | 0.501 | 0.157 | 0.376 | 0.019 |
| *3* | E₂ | 0.892 | 0.336 | 0.472 | 0.089 |
| *4* | E3 | 0.534 | 0.221 | 0.603 | 0.039 |
| *5* | 16-epiE₃ | 0.633 | 0.448 | 0.668 | 0.198 |
| *6* | 2-MeOE₁ | 0.412 | 0.172 | 0.386 | 0.017 |
| *7* | 2-MeOE₂ | 0.493 | 0.154 | 1.044 | 0.042 |
| *8* | 17-epiE₃ | 0.466 | 0.298 | 0.649 | 0.226 |

The Mann-Whitney U test was used for statistical analysis, which was performed using GraphPad Prism software (GraphPad Software, v.4, La Jolla, CA, USA). The following estrogen metabolites were evaluated: estradiol (E2), α-estradiol, 2-methoxyestradiol (2-MeOE2), estrone (E1), 2-methoxyestrone (2-MeOE1), estriol (E3), 16-epiestriol, 17-epiestrol (Table 2). In the group of patients with endometrial cancer, significantly elevated levels of estradiol (E2) (p < 0.0001) and decreased levels of 2-MeOE2 (p < 0.0001) were found compared to the control group. In addition, differences in the levels of α-estradiol and 16-epiestriol were demonstrated (< 0.05).

Considering the pro- and anti-carcinogenic properties of estrogens and their metabolites, the ratio of estradiol to 2-methoxyestradiol was compared in both groups (E2/2-MeOE2 ratio). The assessment was performed using a one-way ANOVA test, followed by Tukey's post-hoc test. In the group of patients with endometrial cancer, the E2 level was almost twice as high as the MeOE2 level (ratio - 1.81, p < 0.0001). The opposite relationship was observed in the control group (ratio - 0.45, p <0.0001). The results of this study indicate that an elevated E2/2-MeOE2 ratio may be used as a potential biomarker for assessing the risk of endometrial cancer.

### B. Determination of hydrogen peroxide in blood serum using the stopped-flow method.

The second stage of the study involved the assessment of hydrogen peroxide (H₂O₂) concentrations. For this purpose, 10 patients (n = 10) aged 59-79 years with diagnosed endometrial cancer were enrolled in the study. The control group consisted of 10 women (n = 10), volunteers aged 39-84, who were qualified for the study by a gynecologist after exclusion of an active proliferative process in the uterine cavity. Blood for testing was collected before surgical treatment and before the implementation of chemotherapy, using sodium heparin. The blood was then centrifuged at 1200 rpm for 10 minutes to separate the plasma from the red blood cells. The plasma was stored at -85°C until analysis.

The concentration of hydrogen peroxide was determined using the stopped-flow method. For this purpose, a microvolume stopped-flow test kit - stopped-flow SX.18MV-R1, which is part of the equipment of the Faculty of Chemistry at the University of Gdańsk, was used.

This method involves determining hydrogen peroxide by measuring the rate of reaction of released carbon monoxide (IV) and, at the same time, determining the concentration of hydrogen peroxide in biological material. The method for determining carbon monoxide (IV) uses sodium pyruvate, which is a direct source of CO₍₂₎ as illustrated by the following reaction equation: pyruvate + H₂O₂→ acetate anion + CO₂+ H₂O. As can be seen from the above reaction, the stoichiometric ratio of carbon monoxide (IV) to hydrogen peroxide is 1:1, so the calculated amount of CO₂ is equivalent to the amount of hydrogen peroxide present in the tested system.

For this purpose, a biosensor previously synthesized by the inventors was used - a coordination compound of chromium (III) with pyridoxamine {cis-[Cr(C₂O₄)(pm)(OH₂)₂]⁺}. The reagent solutions were placed separately in two working syringes, A and B. Syringe A contained the biological material obtained and a 5 mM solution of potassium pyruvate mixed in a 1:1 molar ratio, while syringe B contained a 1 mM solution of the biosensor in a phosphate buffer solution with a pH of 7.4. The solutions are then passed through a mixer (mixing occurs very quickly - 10⁻³ sec. and then through the measuring cell to the return syringe. Filling the return syringe with the solution causes the piston to be pushed out, striking the microswitch. At this point, the flow stops and the measurement begins. The progress of the reaction in the portion of the solution retained in the measuring cell was monitored spectrophotometrically. During the measurement, the change in substrate concentration as a function of time was measured. The described method is based on the assumption of a selective

reaction of α-keto acid pyruvate with hydrogen peroxide, followed by decarboxylation of the intermediate - pyruvic acid - and capture of the released carbon monoxide(IV) by the biosensor - cis-[Cr(C₂ O₄ )(pm)(OH₂)₂]+.

The hydrogen peroxide concentration values were calculated using global analysis, which is based on collecting the results in the form of a set of absorption spectra measured for the entire wavelength range (330-700 nm) characteristic for a given compound within a specified time interval.

The results of the measurements are presented in Table 3.

**Table 3 - Hydrogen peroxide concentration values in a group of patients with endometrial cancer.**

| *Patient* | *H₂O₂ [M = mol*/*dm³]* |
|---|---|
| *1.* | 5.11 · 10⁻⁶M ± 4.21 · 10⁻⁸ |
| *2.* | 8.22 · 10⁻⁶M ± 1.98 · 10⁻⁸ |
| *3.* | 1.54 · 10⁻⁶M ± 7.07 · 10⁻⁸ |
| *4.* | 3.49 · 10⁻⁶M ± 7.33 · 10⁻⁸ |
| *5.* | 9.06 · 10⁻⁶ M ± 6.89 · 10⁻⁸ |
| *6.* | 4.22 · 10⁻⁶M ± 3.78 · 10⁻⁸ |
| *7.* | 5.59 · 10⁻⁶M ± 1.08 · 10⁻⁸ |
| *8.* | 4.79 · 10⁻⁶M ± 2.88 · 10⁻⁸ |
| *9.* | 9.21 · 10⁻⁶M ± 2.46 · 10⁻⁸ |
| *10.* | 9.09 · 10⁻⁶M ± 1.03 · 10⁻⁸ |

In the group of patients with endometrial cancer, hydrogen peroxide was found at a concentration of 5.93 ± 2.46 µmol/L. Using the above method, no hydrogen peroxide was found in the control group. The Mann-Whitney U test was used for statistical analysis.

### Conclusions:

The conducted studies confirm that there are significant differences in the levels of estradiol, 2-methoxyestradiol, and H₂O₂ between endometrial cancer and normal controls. The assessment of the concentrations of the studied biomarkers and their correlation is novel, as no studies have been published to date showing correlations between the concentrations of estradiol metabolites and hydrogen peroxide in patients with endometrial cancer. no studies have been published showing correlations between the concentration of estradiol metabolites and hydrogen peroxide in patients with endometrial cancer.

### Bibliography:

1. Gentry-Maharaj A, Karpinskyj C. Current and future approaches to screening for endometrial cancer. Best Pract Res Clin Obstet Gynaecol. 2020 May;65:79-97.
2. Njoku K, Sutton CJ, Whetton AD, Crosbie EJ. Metabolomic biomarkers for detection, prognosis and identifying recurrence in endometrial cancer. Vol. 10, Metabolites. MDPI AG; 2020. p. 1-28.
3. ACOG Committee Opinion No. 734: The Role of Transvaginal Ultrasonography in Evaluating the Endometrium of Women With Postmenopausal Bleeding. Obstetrics & Gynecology. 2018 May;131(5):e124-9.
4. Schramm A, Ebner F, Bauer E, Janni W, Friebe-Hoffmann U, Pellegrino M, et al. Value of endometrial thickness assessed by transvaginal ultrasound for the prediction of endometrial cancer in patients with postmenopausal bleeding. Arch Gynecol Obstet. 2017 Aug 20;296(2):319-26.
5. Clarke MA, Long BJ, Del Mar Morillo A, Arbyn M, Bakkum-Gamez JN, Wentzensen N. Association of Endometrial Cancer Risk With Postmenopausal Bleeding in Women. JAMA Intern Med. 2018 Sep 1;178(9):1210.
6. Rodriguez AC, Blanchard Z, Maurer KA, Gertz J. Estrogen Signaling in Endometrial Cancer: a Key Oncogenic Pathway with Several Open Questions. Horm Cancer. 2019 Jun 2;10(2-3):51-63.
7. Kamal A, Tempest N, Parkes C, Alnafakh R, Makrydima S, Adishesh M, et al. Hormones and endometrial carcinogenesis. Horm Mol Biol Clin Investig. 2016 Feb 1;25(2):129-48.
8. Lépine J, Audet-Walsh E, Grégoire J, Têtu B, Plante M, Ménard V, et al. Circulating estrogens in endometrial cancer cases and their relationship with tissular expression of key estrogen biosynthesis and metabolic pathways. Journal of Clinical Endocrinology and Metabolism. 2010;95(6):2689-98.
9. Zeleniuch-Jacquotte A, Akhmedkhanov A, Kato I, Koenig KL, Shore RE, Kim MY, et al. Postmenopausal endogenous estrogens and risk of endometrial cancer: results of a prospective study. Br J Cancer. 2001 Apr 5;84(7):975-81.
10. Zhao H, Jiang Y, Liu Y, Yun C, Li L. Endogenous Estrogen Metabolites as Biomarkers for Endometrial Cancer via a Novel Method of Liquid Chromatography-Mass Spectrometry with Hollow Fiber Liquid-Phase Microextraction. Hormone and Metabolic Research. 2014 Apr 10;47(02):158-64.
11. Heidari F, Rabizadeh S, Mansournia MA, Mirmiranpoor H, Salehi SS, Akhavan S, et al. Inflammatory, oxidative stress and anti-oxidative markers in patients with endometrial carcinoma and diabetes. Cytokine. 2019 Aug;120:186-90.
12. Bolton J. Quinoids, quinoid radicals, and phenoxyl radicals formed from estrogens and antiestrogens. Toxicology. 2002 Aug 1;177(1):55-65.
13. Pinto MP, Medina RA, Owen GI. 2-Methoxyestradiol and Disorders of Female Reproductive Tissues. Horm Cancer. 2014 Oct 25;5(5):274-83.
14. Yager JD. Catechol-O-methyltransferase: characteristics, polymorphisms and role in breast cancer. Drug Discov Today Dis Mech. 2012 Jun;9(1-2):e41-6.
15. Salama SA, Kamel M, Awad M, Nasser A Ben, Al-Hendy A, Botting S, et al. Catecholestrogens induce oxidative stress and malignant transformation in human endometrial glandular cells: Protective effect of catechol- O -methyltransferase. Int J Cancer. 2008 Sep 15;123(6):1246-54.
16. Mueck AO, Seeger H. 2-Methoxyestradiol-Biology and mechanism of action. Steroids. 2010 Oct;75(10):625-31.
17. Y 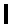 ld 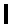 r 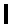 m E, Türkler C, Görkem Ü, 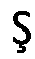 im 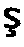 ek ÖY, Y 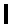 lmaz E, Alada 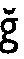 H. The relationship between oxidative stress markers and endometrial hyperplasia: A case-control study. Journal of Turkish Society of Obstetric and Gynecology. 2021 Dec 1;18(4):298-303.

## Claims

1. A set of biomarkers comprising estradiol, 2-methoxyestradiol, and hydrogen peroxide for use in the *in vitro* diagnosis of endometrial cancer in an individual.

2. A method for *in vitro* diagnosis of endometrial cancer in an individual, **characterized in that** it comprises the following steps:
a). in a biological sample taken from an individual, the level of at least two biomarkers selected from a set comprising estradiol, 2-methoxyestradiol, and hydrogen peroxide is determined,
b). the levels of biomarkers in a) are assessed and compared with reference samples,
wherein the endometrial cancer is diagnosed when the level of estradiol is twice as high and the level of 2-methoxyestradiol is twice as low as in the reference sample taken from a healthy individual, and the level of hydrogen peroxide is higher than in the reference sample.

3. The method according to claim 2, **characterized in that** the biological sample is blood plasma.

4. The method according to claim 2, **characterized in that** the levels of estradiol and 2-methoxyestradiol are determined by liquid chromatography coupled with tandem mass spectrometry (LC-MS/MS).

5. The method according to claim 2, **characterized in that** the level of hydrogen peroxide is determined by the stopped-flow method.

6. The method according to claim 2, **characterized in that** the elevated level of hydrogen peroxide is a concentration of hydrogen peroxide in the range of 5.93 ± 2.46 µmol/L.

7. The method according to claim 2, **characterized in that** the level of estradiol is at least twice as high as the level of 2-methoxyestradiol in an individual suffering from endometrial cancer.
